(19) Europäisches Patentamt
European Patent Office
Office européen des brevets

(11) Numéro de publication: **0 044 238**
**B1**

(12) # FASCICULE DE BREVET EUROPEEN

(45) Date de publication du fascicule du brevet: **01.02.84**

(51) Int. Cl.³: **C 07 D 501/36,** C 07 D 501/46, A 61 K 31/545 // C07D277/20

(21) Numéro de dépôt: **81401027.8**

(22) Date de dépôt: **26.06.81**

(54) **Dérivés de céphalosporines sulfoxydes, procédé de préparation et compositions pharmaceutiques en contenant.**

(30) Priorité: **30.06.80 FR 8014512**
**03.03.81 FR 8104242**
**03.03.81 FR 8104243**

(43) Date de publication de la demande:
**20.01.82 Bulletin 82/3**

(45) Mention de la délivrance du brevet:
**01.02.84 Bulletin 84/5**

(84) Etats contractants désignés:
**AT BE CH DE FR GB IT LI LU NL SE**

(56) Documents cités:
**EP - A - 0 002 605**
**EP - A - 0 005 830**
**EP - A - 0 029 966**
**FR - A - 2 426 695**
**FR - A - 2 465 737**

**Le dossier contient des informations techniques présentées postérieurement au dépôt de la demande et ne figurant pas dans le présent fascicule.**

(73) Titulaire: **SANOFI, société anonyme, 40, Avenue George V, F-75008 Paris (FR)**

(72) Inventeur: **Labeeuw, Bernard, 49 Lotissement des Genêtes avenue des Moulins, F-34000 Montpellier (FR)**
Inventeur: **Salhi, Ali, 639 rue Valène, F-34980 St. Gely du Fesc (FR)**

(74) Mandataire: **Combe, André et al, CABINET BEAU DE LOMENIE 55 rue d'Amsterdam, F-75008 Paris (FR)**

EP 0 044 238 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Dérivés de céphalosporines sulfoxydes, procédé de préparation et compositions pharmaceutiques en contenant

La présente invention concerne de nouveaux sulfoxydes de céphalosporines, un procédé pour leur préparation et des compositions pharmaceutiques renfermant lesdits sulfoxydes de céphalosporines en tant qu'ingrédients actifs.

Plus particulièrement, la présente invention se réfère à de nouvelles céphalosphorines sulfoxydes ayant en position 3 du noyau céphème soit un groupement dihydro-4,5-hydroxy-6-oxo-5-1,2,4-triazinyl-thiométhyle, soit un groupement pyridinium méthyle éventuellement substitué.

Le brevet belge 866 038 décrit une série de sulfoxydes et de sulfones de céphalosporines des formule générale:

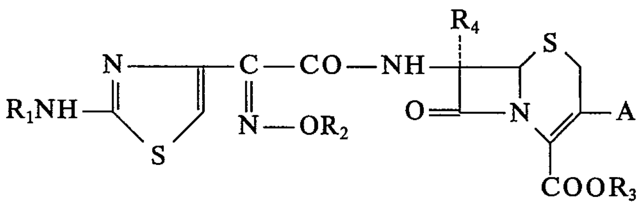

(I)

Ce brevet cite parmi les radicaux indiqués pour A des groupes $CH_2S—R_5$ où $R_5$ peut être triazinyle éventuellement substitués et plus particulièrement des composés de formule I où:

$R_1 = H$;

$R_2 = H$;

$R_3 = H$;

$R_4 = H$;

$X = SO$;

A =

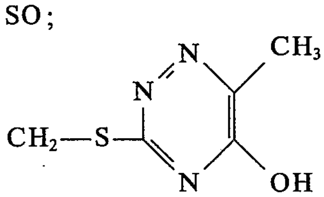

et

$R_1 = H$;

$R_2 = CH_2—CH{=}CH_2$;

$R_3$ =

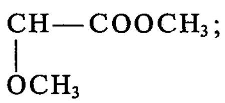

$R_4 = H$;

$X = SO$;

A = 

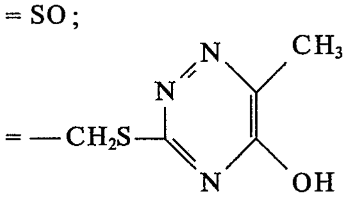

sans toutefois les décrire d'une façon spécifique.

Les céphalosporines de formule I sont censées posséder d'une façon générale une très forte activité antibactérienne contre les bactéries gram positives et gram négatives et être très efficaces contre les staphylocoques producteurs de pénecillinase.

La demande de brevet européen 5830 décrit un groupe de céphalosporines de formule générale:

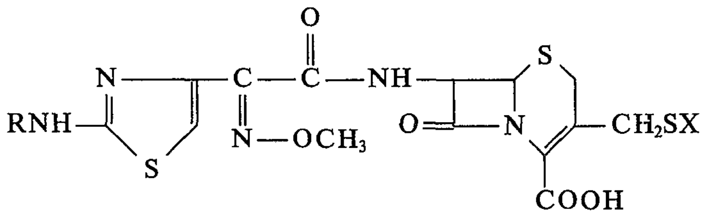

(II)

dans laquelle X représente en particulier

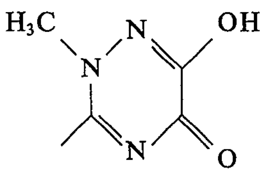

Les céphalosporines de formule II sont présentées comme des antibiotiques à large spectre agissant sur toutes les bactéries gram positives et gram négatives et particulièrement sur les staphylocoques producteurs de β-lactamases.

Enfin, d'autres céphalosporines ont été décrites dans les brevets français 2 465 735 et 2 426 695.

On a trouvé maintenant que certaines céphalosporines

S-oxydes substituées en 3 par un groupe:

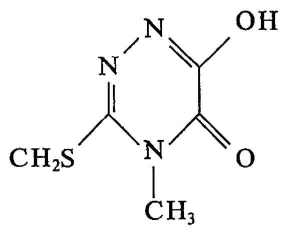

ou par un groupe pyridinium méthyl éventuellement substitué par un hydroxyle, un carboxyle ou un groupe carboxamide montrent un profil bactérien complètement différent de celui des composés décrits dans le brevet belge 866 038 ou la demande de brevet européen 5830.

On a trouvé que lesdites céphalosporines 1-oxydes possèdent une activité remarquable sur les bactéries gram négatives y compris celles productrices de β-lactamases et tout particulièrement sur les ente'robactéries alors qu'elles sont très faiblement actives ou pratiquement inactives sur les staphylocoques producteurs ou non de β-lactamases.

Ainsi, la présente invention a pour objet de nouvelles céphalosphorines répondant à la formule générale:

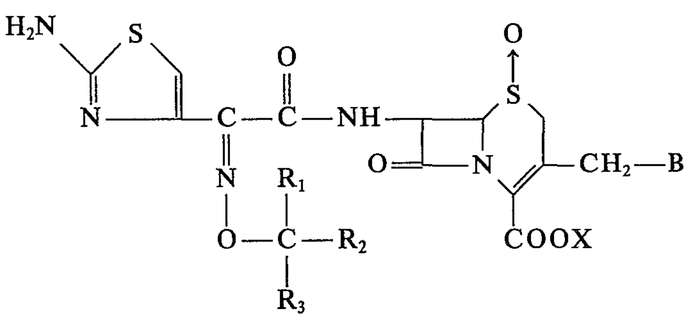

(III)

dans laquelle

| | |
|---|---|
| $R_1$ | représente l'hydrogène ou un groupe COO X' |
| $R_2$ et $R_3$ | qui peuvent être identiques ou différents, représentent chacun l'hydrogène ou un groupe alkyle inférieur<br>ou bien |
| $R_2$ et $R_3$ | représentent ensemble un radical 1,3-propylène ou 1,4-butylène |
| X et X' | qui peuvent être identiques ou différents représentent de l'hydrogène, un cation ou un ester ou hémiacétal facilement hydrolysable ou métaboliquement labile et pharmaceutiquement acceptables |

B désigne un groupe

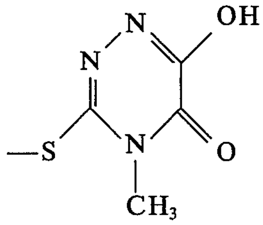

ou encore un groupe pyridinium

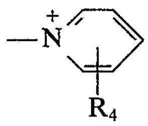

dans lequel $R_4$ représente un hydroxy, un groupe $-COOH$ ou un groupe $-CO-NH_2$.

Dans la présente demande:

– Le terme »alkyl inférieur« tel qu'utilisé ici dédigne le radical d'un hydrocarbure aliphatique saturé contenant jusqu'à 3 atomes de carbone, à savoir méthyle, éthyle, propyle et isopropyle.
– Le terme »cation« tel qu'utilisé ici désigne un ion alcalin ou alcalino-terreux de préférence les ions sodium, potassium ou calcium ou bien le dérivé »ammonium« résultant par protonation d'une amine organique pharmaceutiquement acceptable telle que l'éthylènediamine, l'éthanolamine, la triéthanolamine et similaires pour former des sels d'addition.
– Le terme ester ou hémiacétal facilement hydrolysable ou métaboliquement labile et pharmaceutiquement acceptable désigne des radicaux tels que phtalidyle, pivaloyloxyméthyle, acétoxyméthyle, éthoxycarbonyloxyméthyle, 1-(éthoxycarbonyloxy)éthyle, acétonyle, $\alpha$-méthoxy-$\alpha$-carbométhoxyméthyle, carbométhoxyméthyle, carbéthoxyméthyle et similaires.

L'invention concerne également des procédés de préparation des composés de formule (III).

Un premier procédé comprend les étapes schématisées ci-après:

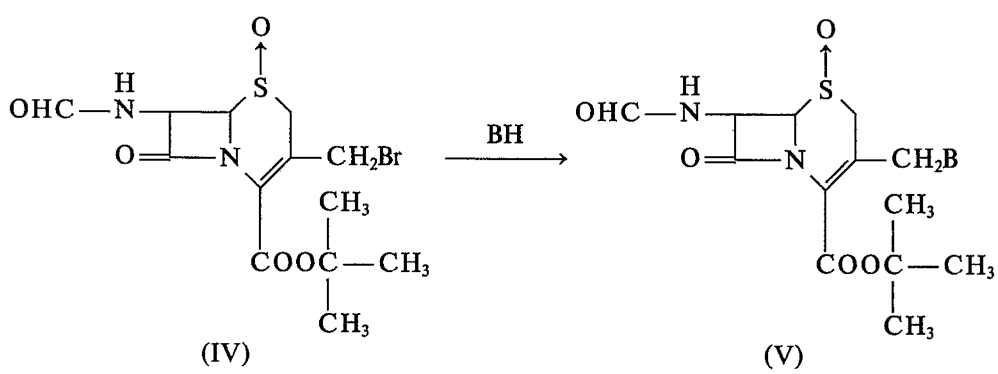


(IV) (V)

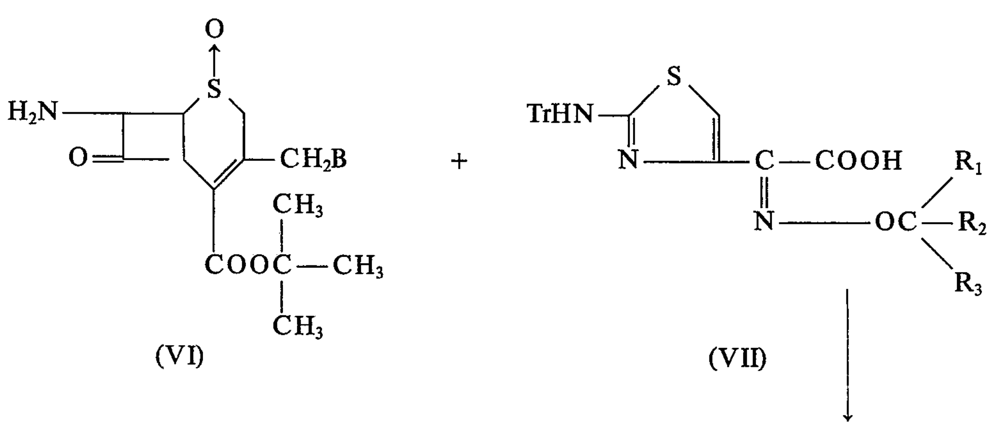

(VI) (VII)

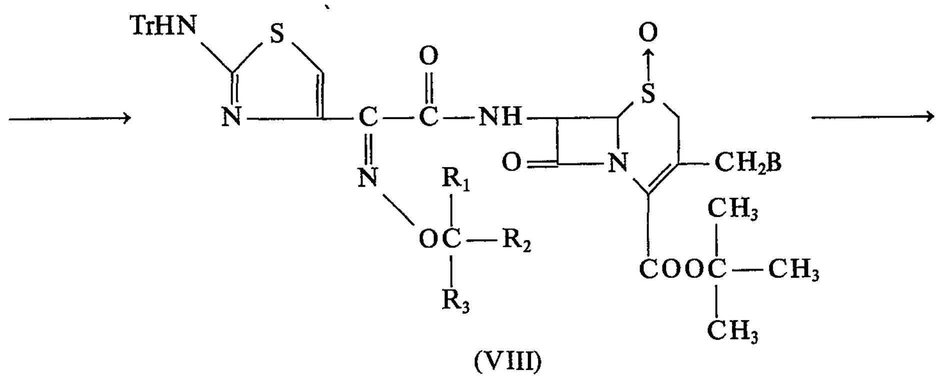

(III)

(VIII)

Tr = trityle

On part du sulfoxyde (IV) qui par action du composé BH ou B en solution dans le diméthylformamide en présence d'une base comme la triéthylamine conduit au composé convenablement substitué en position 3 (V). On libère le groupe amino de celui-ci par action du chlorure de thionyle en solution et on isole le composé (VI) sous forme de chlorhydrate. Celui-ci est acylé par le composé (VII).

Avant d'effectuer la réaction d'acylation, il est souhaitable de substituer le groupe amino de l'acide par un groupe protecteur facile à éliminer ultérieurement. On peut utiliser les groupes habituellement utilisés en synthèse organique pour la protection des groupes aminés et en particulier le groupe trityle.

Pour effectuer la réaction d'acylation, il est nécessaire de procéder à l'activation du groupe carboxyle du composé (VII) de préférence par transformation en anhydride à l'aide d'un carbodiimide en général le dicyclohexylcarbodiimide.

La réaction d'activation est effectuée au sein d'un solvant organique convenable tel que le tétrahydrofuranne à une température comprise entre 0 et 50°C et de préférence à température ambiante. La réaction d'activation est éventuellement facilitée par addition d'un dérivé hydroxylé tel que l'hydroxy-1 benzotriazole.

La solution du réactif d'acylation ainsi obtenu, débarrassée par filtration de la dicyclohexylurée formée, est ajoutée à une solutin du composé (VI) dans un solvant tel que le diméthylformamide. L'addition des deux réactifs peut aussi s'effectuer dans l'ordre inverse.

Après la réaction d'acylation, le groupe protecteur sur l'amine et l'ester tertiobutylique sont éliminés par un procédé connu, en particulier par hydrolyse en milieu acide en utilisant un acide organique tel que l'acide formique ou l'acide trifluoroacétique.

En ce qui concerne les matières premières de la réaction, les composés (IV) et le composé (VII) ainsi que ses dérivés dans lesquels le groupe aminé est bloqué par un groupe protecteur sont connus.

Une variante de ce procédé consiste à acyler tout d'abord l'amino-7 bromométhyl-3 céphème-3 carboxylate de tertiobutyle-4 (IX) par l'acide (VII). On opère ainsi qu'il est indiqué pour l'acylation des composés (VI).

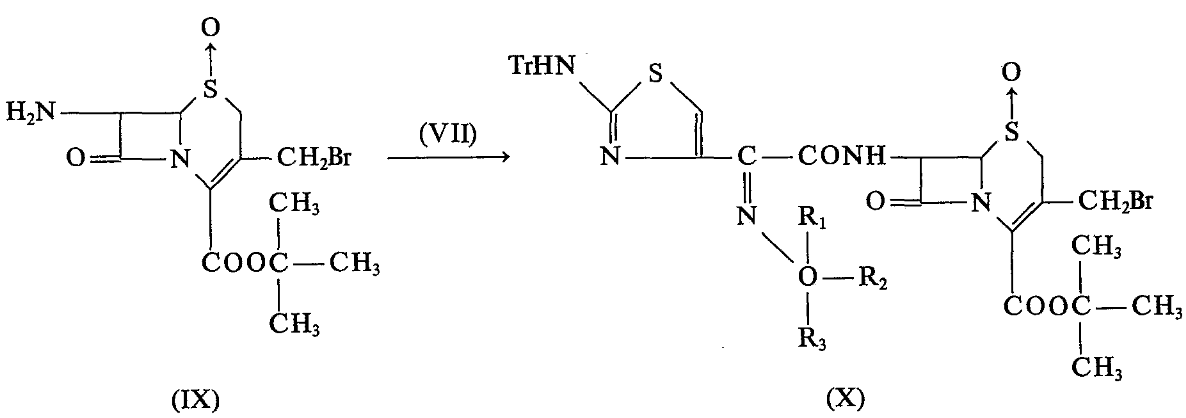


(IX) (X)

B

(VIII) (III)

Par action sur le composé (X) ainsi obtenu d'un thiol BH ou d'une amine B on obtient le composé (VIII) correspondant. On opère dans les conditions indiquées pour l'obtention des composés (V).

Comme indiqué précédemment, les composés (VIII) traités par un acide fort conduisent aux composés (III).

Les composés (III) de l'invention dans lesquels X et autre que H s'obtiennent à partir des composés (III)

dans lesquels X est H par des réactions connues en elles-mêmes.

Ainsi les sels minéraux sont obtenus par action sur les composés (III) dans lesquels X est H d'une base minérale telle que la soude ou la potasse ou le bicarbonate de sodium en quantité équimoléculaire; la réaction de salification est réalisée dans un solvant tel que l'eau ou l'éthanol et le sel obtenu est isolé par évaporation de la solution.

Les sels de bases organiques sont obtenus par action, sur une solution de l'acide (III X=H) dans un solvant ou un mélange de solvants convenable, d'une quantité équimoléculaire de la base organique. Le sel est isolé par précipitation avec l'éther.

Les esters sont obtenus par les procédés connus d'estérification; par exemple on utilisera avantageusement l'action d'un dérivé halogéné sur un sel tel que le sel de sodium de l'acide; on réalisera de préférence la réaction dans un solvant capable de dissoudre le dérivé acide de départ par exemple dans le diméthylformamide.

Les isomères de forme syn et anti s'obtiennent par un choix convenable des réactifs.

Les exemples suivants permettent de mieux comprendre la portée de l'invention.

Ainsi qu'il est habituel dans cette famille de composés, les produits suivant l'invention ne présentent pas de point de fusion net mais seulement des points de décomposition ne permettant pas de les caractériser.

Les produits seront donc caractérisés par leur spectre de résonance magnétique nucléaire enregistré à 60 MHz, l'étalon interne étant l'hexaméthyldisiloxanne.

Les abréviations suivantes seront utilisées:

- S: singulet
- D: doublet
- D de D: doublet de doublet
- S. el: singulet élargi
- M: multiplet
- AB: système AB
- J: représente la constante de couplage.

De plus les microanalyses élémentaires ont été effectuées dans chaques cas et sont en accord avec les formes indiquées.

## Exemple 1

Acide [(amino-2 thiazolyl-4)-2 méthoxyimino-2 acétamido]-7 [(hydroxy-6 méthyl-4 oxo-5 dihydro-4,5 triazin-1,2,4 yl)-3 thiométhyl]-3 céphème-3 carboxylique-4 S-oxydr-1

Isomère syn (CM 40 365)

$R_1 = R_2 = R_3 = H$;

B = 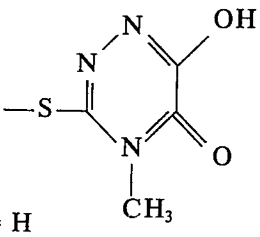 (III)

X = H

a) Formamido-7 [(hydroxy-6 méthyl-4 oxo-5 dihydro-4,5 triazin-1,2,4 yl)-3 thiométhyl]-3 céphème-3 carboxylate de tertio-butyle-4 S-oxyde

B = 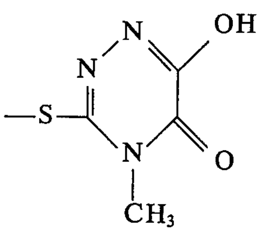 (V)

A une solution de 5 g de formamido-7 bromométhyl-3 céphème-3 carboxylate de tertiobutyle-4 S-oxyde-1 dans 60 ml de diméthylformamide, on ajoute 2,13 g d'hydroxy-6 mercapto-3 méthyl-4 oxo-5 dihydro-4,5 triazine-1,2,4 puis une solution de 1,78 ml de triéthylamine dans 6,65 ml de diméthylformamide.

On agite pendant 16 heures à température ambiante, puis on dilue par addition de chlorure de méthylène et précipite le produit attendu par addition lente d'éther isopropylique.

Poids: 6,5 g.

Spectre de RMN (en solution dans le diméthylsulfoxyde deutérié):

1H à 12,2 ppm (OH, S. el) – 1H à 9,00 ppm (NH CO, D, J=9 Hz) – 1H à 8,10 ppm (H CO, S) – 1H à 5,75 ppm ($H_7$, D de D, $J_1$=9 Hz, $J_2$=4 Hz) – 1H à 5,07 ppm ($H_6$, D, J=4 Hz) – 2H à 5,00 ppm ($CH_2$ en 2, AB, $J_{AB}$=13 Hz) – 2H à 3,63 ppm ($CH_2$–S, S. el) – 3H à 3,27 ppm (N–$CH_3$, S) –

9H à 1,45 ppm 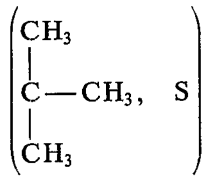

b) Amino-7 [(hydroxy-6 méthyl-4 oxo-5 dihydro-4,5 triazin-1,2,4 yl)-3 thiométhyl]-3 céphème-3 carboxylate de tertio-butyle-4 S-oxyde-1

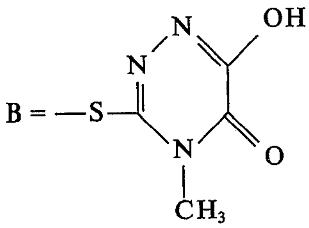

(VI)

A une solution de 1 g du produit précédent dans 20 ml de méthanol, on ajoute 0,295 ml de triéthylamine puis 2,47 ml de chlorure de thionyle en maintenant la température du mélange entre 2 et 10°C. On concentre rapidement sous vide poussé à température ambiante. On ajoute de l'éther et essore le solide précipité. On sèche sous vide en présence d'anhydride phosphorique.

On obtient finalement 1,1 g du produit attendu contenant du chlorhydrate de triéthylamine.

Spectre de RMN (en solution dans le diméthylsulfoxyde deutérié):

2H à 4,70 ppm ($H_6$ et $H_7$, AB, $J_{AB}$=4 Hz) – 2H à 4,02 ppm ($CH_2S$, AB, $J_{AB}$=13 Hz) – 2H à 3,72 ppm ($CH_2$ en 2, AB, $J_{AB}$=17 Hz) – 3H à 3,25 ppm

(N–$CH_3$, S) – 2H à 280 ppm ($NH_2$, S. el) –

9H à 1,45 ppm 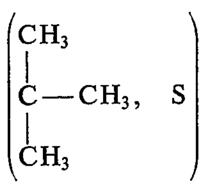

c) [(tritylamino-2 thiazolyl-4)-2 méthoxyimino-2 acétamido]-7 [hydroxy-6 méthyl-4 oxo-5 dihydro-4,5 triazin-1,2,4 yl)-3 thiométhyl]-3 céphème-3 carboxylate de tertiobutyle-4 S-oxyde-1 isomère syn

$R_1 = R_2 = R_3 = H$;

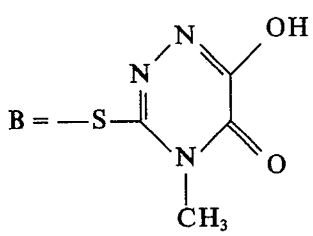

(VIII)

Pour préparer l'ester activé, on agite pendant 1 heures à température ambiante, le mélange de 1,1 g d'acide (tritylamino-2 thiazolyl-4)-2 méthoxyimino-2 acétique isomère syn, 0,516 g de dicyclohexyl carbodiimide et 0,338 g d'hydroxy-1 benzotriazole dans 5 ml de diméthylformamide. Après achèvement de la réaction, on filtre la dicyclohexylurée formée.

La solution ainsi obtenue est ajoutée à une solution de 1 g du composé obtenu au paragraphe b) et 0,29 ml de triéthylamine dans 5 ml de diméthylformamide.

On laisse le mélange réactionnel 48 heures sous agitation à température ambiante puis on évapore le diméthylformamide sous vide. On reprend le résidu dans le chlorure de méthylène, lave la solution avec une solution aqueuse d'acide chlorhydrique 1 N puis avec de l'eau et enfin avec une solution aqueuse de bicarbonate de sodium. On sèche la solution sur sulfate de magnésium et évapore le solvant à siccité sous vide.

On chromatographie le résidu sur une colonne de gel de silice (120 g) et on élue avec un mélange chlorure de méthylène/méthanol 96—4 (vol/vol).

On obtient ainsi 0,6 g du prodiut attendu.

Spectre de RMN (en solution dans le diméthylsulfoxyde deutérié):

1 H à 8,80 ppm (NH CO, D, J=9 Hz) — 1 H à 8,70 ppm (NH-Trit, S) — 15H à 7,27 ppm (H trityle, S) — 1 H à 6,75 ppm (H thiazole, S) — 1 H à 6,71 ppm ($H_7$, D de D, $J_1$=9 Hz, $J_2$=4 Hz) — 1 H à 4,88 ppm ($H_6$, D, J=4 Hz) — 2H à 4,05 ppm ($CH_2S$, AB, $J_{AB}$=13 Hz) — 5H à 3,75 ppm ($CH_2$ en 2 et $OCH_3$, S. el) — 3 H à 3,21 ppm (—N $CH_3$, S) —

9H à 1,45 ppm 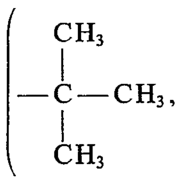 S

d) CM 40365

On agite pendant 30 minutes à température ambiante 0,6 g du produit obtenu précédemment avec 6 ml d'acide trifluoracétique. On concentre sous vide à un volume de 2 ml et ajoute de l'éther isopropylique. On essore le solide précipité (0,35 g).

Spectre de RMN (en solution dans le diméthylsulfoxyde deutérié):

1H à 8,78 ppm (NHCO, D, J=9 Hz) — 1H à 6,79 ppm ( H thiazole, S) — Massif entre 4 et 7 ppm déplaçable par $D_2O$ ($NH_2$, COOH, OH) — 1H à 5,80 ppm ($H_7$, D de D, $J_1$=9 Hz, $J_2$=4 Hz) — 1H à 4,92 ppm ($H_6$, D, J=4 Hz) — 2H à 4,10 ppm ($CH_2S$, AB, $J_{AB}$=14 Hz) — 5H à 3,80 ppm ($CH_2$ en 2 et $OCH_3$, S. el) — 3H à 3,24 ppm ($NCH_3$, S).

Exemple 2

Acide [(amino-2 thiazolyl-4)-2 (carboxy-2 propyl-2 oximino)-2 acétamido]-7 [(hydroxy-6 méthyl-4 oxo-5 dihydro-4,5 triazin-1,2,4 yl)-3 thiométhyl]-3 céphème-3 carboxylique-4 S-oxyde-1 isomère syn (CM 40419).

$R_1$ = COOH

$R_2$ = $R_3$ = $CH_3$

X = H

B = 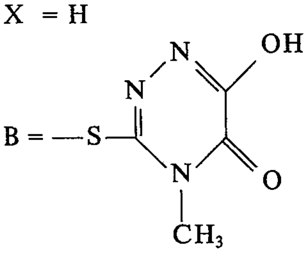 (III)

a) [(tritylamino-2 thiazolyl-4)-2 (t-butoxycarbonyl-2 propyl-2 oximino)-2 acétamido]-7 brométhyl-3 céphème-3 carboxylate de tertiobutyle-4 S-oxyde-1 isomère syn

$R_1$ = 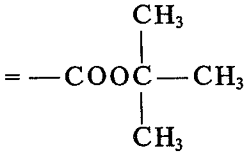 (X)

$R_2 = R_3 = CH_3$

A une solution de 5 g de chlorhydrate d'amino-7 bromométhyl-3 céphème-3 carboxylate de tertiobutyle-4 S-oxyde-1 dans 90 ml de chlorure de méthylène, on ajoute 1,72 ml de triéthylamine, 7,57 g d'acide (tritylamino-2 thiazolyl-4)-2 (t-butoxycarbonyl-2 propyl-2 oximino)-2 acétique, 2,84 g de dicyclohexylcarboiimide et 0,1 g d'hydroxy benzotriazole. On agite le mélange pendant 15 heures à température ambiante puis on filtre la dicyclohexylurée formée.

Après évaporation du solvant, on chromatographie le résidu sur une colonne de gel de silice (250 g). En éluant avec un mélange hexane-acétate d'éthyle 50—50 (vol/vol), on obtient 4,3 g du produit attendu.

Spectre de RMN (en solution dans le diméthylsulfoxyde deutérié):

1H à 8,70 ppm (NH-Trit, S) — 1H à 8,07 ppm (NH—CO, D, J=9 Hz) — 15H à 7,25 ppm (H Trit, S) — 1H à 6,72 ppm (H thiazole, S) — 1H à 5,88 ppm ($H_7$, D de D, $J_1$=9 Hz, $J_2$=4 Hz) — 1H à 4,96 ppm ($H_6$, D, J=4 Hz) — 2H à 4,50 ppm ($CH_2Br$, AB, $J_{AB}$=12 Hz) — 2H à 3,77 ppm ($CH_2$ en 2, S. el) —

9H à 1,45 ppm 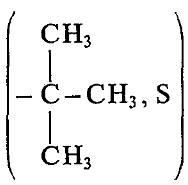 — 6H à 1,37 ppm 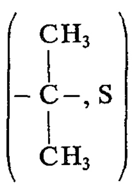 —9H à 1,27 ppm 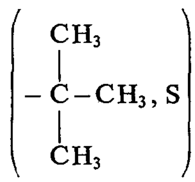.

b) [(tritylamino-2 thiazolyl-4)-2 (t-butoxycarbonyl-2 propyl-2 oximino)-2 acétamido]-7 [(hydroxy-6 méthyl-4 oxo-5 dihydro-4,5 triazin-1,2,4 yl)-3 thiométhyl]-3 céphème-3 carboxylate de tertiobutyle-4 S-oxyde-1 isomère syn

$R_1$ = 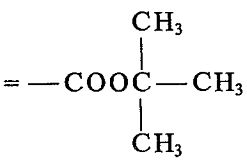 B = 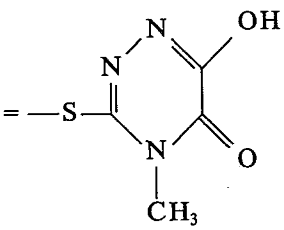 (VIII)

$R_2 = R_3 = CH_3$

A une solution de 1 g du produit obtenu précédemment dans 12 ml de diméthylformamide, on ajoute 0,195 g du sel de sodium de l'hydroxy-6 mercapto-3 méthyl-4 oxo-5 dihydro-4,5 triazine-1,2,4 puis 0,15 ml de triéthylamine. On agite 3 heures à température ambiante puis on précipite le produit attendu par addition d'éther isopropylique. Poids 0,8 g.

Spectre de RMN (en solution dans le diméthylsulfoxyde deutérié):

1H à 8,70 ppm (NH trit, S) — 1H à 8,00 ppm (NH—CO, D, J=9 Hz) — 15H à 7,26 ppm (Trit, S) — 1H à 6,75 ppm (H thiazole, S) — 1H à 5,82 ppm ($H_7$, D de D, $J_1$=9 Hz, $J_2$=4 Hz) — 1H à 4,96 ppm ($H_6$, D, J=4 Hz) — 2H à 4,10 ppm ($CH_2S$, AB, $J_{AB}$=13 Hz) — 2H à 3,82 ppm ($CH_2$ en 2, S. el) — 3H à 3,25 ppm (N—$CH_3$, S) —

9H à 1,45 ppm 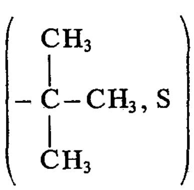 — 6H à 1,35 ppm 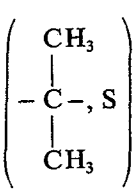 —9H à 1,28 ppm 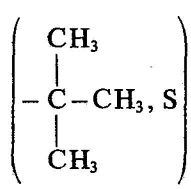.

c) CM 40419

On agite pendant 30 minutes à température ambiante une solution de 0,89 g du composé obtenu ci-dessus dans 9 ml d'acide trifluoracétique. On concentre la solution sous vide jusqu'à un volume de 5 ml et ajoute de l'éther isopropylique jusqu'à précipitation. On essore le précipité et on le redissout dans 800 ml d'un mélange acétone-éthanol 50—50 (vol/vol). On concentre sous vide jusqu'à 50 ml puis on essore le précipité (0,450 g).

Spectre de RMN (en solution dans le diméthylsulfoxyde):

1H à 8,21 ppm (NH—CO, D, J=9 Hz) — 4H à 7,22 ppm ($NH_2$, COOH, OH, S. el) — 1H à 6,77 ppm (H thiazole, S) — 1H à 5,90 ppm ($H_7$, D de D, $J_1$=9 Hz), $J_2$=4 Hz) — 1H à 4,91 ppm ($H_6$, D, J=4 Hz) — 2H à 4,15 ppm ($CH_2S$, AB, $J_{AB}$=13 Hz) — 2H à 3,74 ppm ($CH_2$ en 2, S. el) — 3H à 3,22 ppm (N—$CH_3$, S) —

6H à 1,38 ppm ( 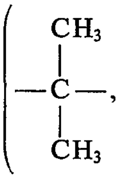 , S) .

En opérant comme dans l'exemple 2 mais en faisant varier l'acide VII utilisé dans la première étape, on obtient de façon analogue les composés (III) réunis dans le tableau suivant.

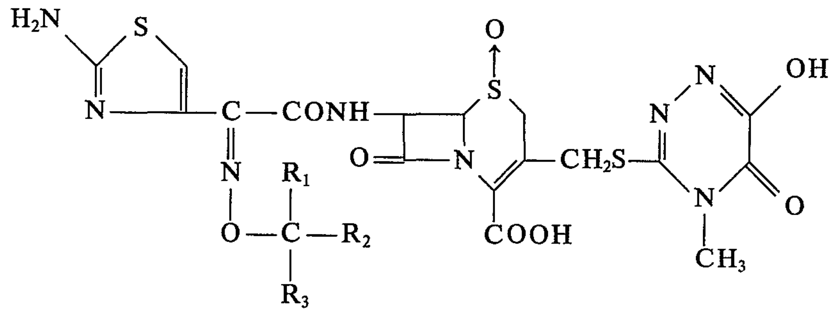

| N code CM | $R_1$ | $R_2$ | $R_3$ | Spectre de RMN |
|---|---|---|---|---|
| 40513 | COOH | H | H | 1H à 8,26 ppm (NH—CO, D, J=8,5 Hz) — 1H à 6,92 ppm (H thiazole, S) — 5H à 6,40 ppm (H échangeables, S. e.) — 1H à 5,92 ppm ($H_7$, D de D, $J_1$=8,5 Hz) — 1H à 4,94 ppm ($H_6$, D, J=4,5 Hz) — 1H à 4,60 ppm ($OCH_2$, S) — 1H à 4,27 ppm (CH S-triazine, A de AB, $J_{AB}$=15 Hz) — 1H à 3,97 ppm (CHS-triazine, B de AB, $J_{AB}$=15 Hz) — 2H à 3,82 ppm ($CH_2$—S→O, S. e.) — 3H à 3,25 ppm ($CH_3$—N, S). |
| 40473 | COOH | $-CH_2CH_2CH_2-$ | | 1H à 8,75 ppm (NH CO, D, J=8,5 Hz) — 5H à 8,0 ppm (H échangeables, S. e.) — 1H à 6,91 ppm (H thiazole, S) — 1H à 5,99 ppm ($H_7$, D de D, $J_1$=8,5 Hz, $J_2$=4,5 Hz) — 1H à 5,00 ppm ($H_6$, D, J=4,5 Hz) — 1H à 4,3 ppm ($CH_2S$, A de AB, $J_{AB}$=15 Hz) — 1H à 4,0 ppm ($CH_2S$, B de AB, $J_{AB}$=15 Hz) — 2H à 3,85 ppm ($CH_2S$→O, S. e.) — 3H à 3,28 ppm (N—$CH_3$, S) — 6H entre 1,5 et 2,6 ppm (cyclobutyle, M) |

Fortsetzung

| N code CM | $R_1$ | $R_2$ | $R_3$ | Spectre de RMN |
|---|---|---|---|---|
| 40471 | COOH | $-CH_2CH_2CH_2CH_2-$ | | 1H à 8,50 ppm (NH CO, D, J=8,5 Hz) — 5H à 8,40 ppm (H échangeables, S) — 1H à 6,89 ppm (H thiazole, S) — 1H à 6,0 ppm ($H_7$, D de D, $J_1$=8,5 Hz, $J_2$=4,5 Hz) — 1H à 5,96 ppm ($H_6$, D, J=4,5 Hz) — 1H à 4,30 ppm ($CH_2S$, A de AB, $J_{AB}$=15 Hz) — 1H à 4,0 ppm ($CH_2S$, B de AB, $J_{AB}$=15 Hz) — 8H entre 1,3 et 2,4 ppm (cyclopentyle, M). |

## Exemple 3

Trifluoracétate de l'acide [(amino-2 thiazolyl-4)-2 carboxyméthoxy imino-2acétamido]-7 (pyridinio-1 méthyl)-3 céphème-3 carboxylique-4 S-oxyde-1 isomère syn (CM 40512)

a) [tritylamino-2 thiazolyl-4)-2 t-butoxycarbonyl-méthoxyimino-2 acétamido]-7 bromométhyl-3 céphème-3 carboxylate de tertiobutyle-4 S-oxyde-1 isomère syn (X)

$R_1$ = 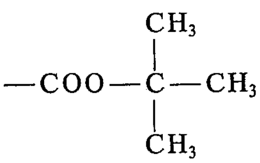

$R_2 = R_3 = H$

A une solution de 3,26 g de chlorhydrate d'amino-7 bromométhyl-3 céphème-3 carboxylate de tertiobutyle-4 S-oxyde-1 dans 58 ml de chlorure de méthylène anhydre, on ajoute 1,12 ml de triéthylamine, 4,86 g d'acide (tritylamino-2 thiazolyl-4)-2 t-butoxycarbonyl méthoxyimino-2 acétique isomère syn, 1,84 g de dicyclohexyl-carbodiimide et 0,1 g d'hydroxy-1 benzotriazole.

On agite à température ambiante pendant 3 heures 30 minutes puis on filtre la dicyclohexylurée formée. On concentre le solvant à environ 10 ml sous vide, puis on chromatographie sur colonne de gel de silice.

Par élution avec un mélange hexane-acétate d'éthyle 60—40 (vol/vol) on obtient le produit attendu (2,8 g).

Spectre de RMN:

1H à 8,75 ppm (NH-Trit, S) — 1H à 8,57 ppm (NH—CO, D, J=8,5 Hz) — 15H à 7,28 ppm (H aromatiques, S) — 1H à 6,82 ppm (H thiazole, S) — 1H à 5,84 ppm ($H_7$, D de D, $J_1$=8,5 Hz, $J_2$=4,5 Hz) — 1H à 4,98 ppm ($H_6$, D, J=4,5 Hz) — 4H à 4,50 ppm ($CH_2Br$ et $OCH_2$, S) — 2H à 3,72 ppm ($CH_2S{\rightarrow}O$, S. e.) —

9H à 1,44 ppm 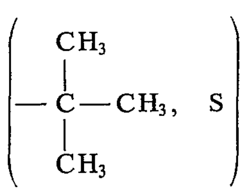 — 9H à 1,35 ppm 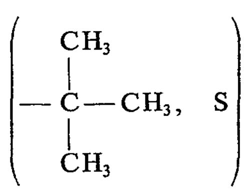.

b) Bromure de [(tritylamino-2 thiazolyl-4)-2 t-butoxy-carbonyl méthoxyimino-2 acétamido]-7 (pyridinio-1 méthyl)-3 céphème-3 carboxylate de tertiobutyle-4 S-oxyde-1 isomère syn (VIII)

$R_1$ = 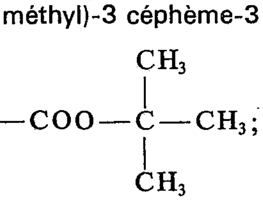 ; B = 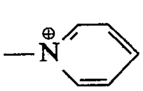

$R_2 = R_3 = H$

On maintient pendant 16 heures à 0°C la solution de 0,4 g du produit obtenu ci-dessus dans 4 ml de pyridine. On évapore la pyridine à siccité sous vide puis on reprend le résidu dans le chlorure de méthylène.

On lave la solution avec une solution diluée d'acide chlorhydrique (pH = 2). On sépare la phase organique, sèche sur sulfate de magnésium et concentre la solution jusqu'à 1 ml. On ajoute 10 ml d'éther et essore le précipité formé (0,350 g).

c) CM 40512

On laisse pendant 30 minutes à température ambiante la solution de 0,310 g du produit obtenu au paragraphe b) dans 3,1 ml d'acide trifluoracétique. On concentre sous vide à 2 ml et précipite le produit par addition d'éther isopropylique. On essore le solide et sèche sous vide.

Le solide est purifié par agitation dans 66 ml d'éthanol à 90° qui fournit 0,100 g de CM 40512. Par traitement sur charbon de la solution et concentration à patit volume, on obtient encore 0,030 g du produit attendu.

Spectre de RMN:

2H à 9,20 ppm ($H_2$, pyridine, M) — 2H à 8,63 ppm (NH CO et $H_4$, pyridine, M) — 2H à 8,15 ppm ($H_3$, pyridine, M) — 3H à 7,23 ppm ($NH_3^+$, S. e.) — 1H à 6,85 ppm (H thiazole, S) — 1H à 5,82 ppm ($H_7$, D de D, $J_1$=8,5 Hz, $J_2$=4,5 Hz) — 2H à 5,55 ppm ($CH_2N^+$, AB, $J_{AB}$=15 Hz) — 1H à 4,98 ppm ($H_6$, D, J= 4,5 Hz) — 2H à 3,52 ppm ($CH_2S{\rightarrow}O$, S. e.).

Exemple 4

Trifluoracétate de l'acide [(amino-2 thiazolyl-4)-2 (carboxy-1 éthyl-1 oxyimino)-2 acétamido]-7 (pyridinio-1 méthyl)-3 céphème-3 carboxylique-4 S-oxyde-1 isomére syn (CM 40678)

a) Acide (tritylamino-2 thiazolyl-4)-2 (butoxycarbonyl-1 éthyl oxyimino)-2 acétique

A la solution de 10 g d'acide (tritylamino-2 thiazolyl-4)-2 hydroxyimino-2 acétique isomère syn dans 110 ml de dioxanne et 0,72 ml d'eau, on ajoute en 15 minutes sous atmosphère d'azote et en refroidissant 6 g de tertiobutylate de potassium dissous dans 50 ml de dioxanne. Après la fin de l'addition, on poursuit l'agitation à température ambiante pendant l'heure puis on ajoute la solution de 6,35 g de bromo-2 propionate de t-butyle dans 10 ml de dioxanne. On agite 2 heures à température ambiante puis on ajoute 0,6 ml d'acide acétique et évapore le solvant sous vide.

On reprend le résidu dans 200 ml de chlorure de méthylène et lave la solution organique avec 100 ml d'une solution diluée d'acide chlorhydrique (pH = 2,5). On sépare la phase aqueuse et on la réextrait 2 fois avec 150 ml de chlorure de méthylène. On réunit les extraits organiques, sèches sur sulfate de magnésion et concentre à 30 ml.

On chromatographie sur colonne de gel de silice (500 g). En éluant avec le mélange chloroforme-méthanol 95—5 (vol/vol), on obtient 8,4 g du produit attendu. Fk: 150°C; (décomposition).

b) [(tritylamino-2 thiazolyl-4)-2 (t-butoxycarbonyl-1 éthyl-1 oxyimino)-2 acétamido]-7 bromométhyl-3 céphème-3 carboxylate de t-butyle-4 S-oxyde-1 isomère syn

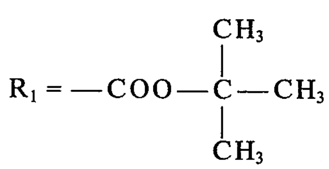

(X)

$R_2 = H$

$R_3 = CH_3$

On opère comme dans l'exemple 3a) en remplaçant l'acide (tritylamino-2 thiazolyl-4)-2 t-butoxycarbonylméthoxyimino-2 acétique par une quantité équivalente d'acide (tritylamino-2 thiazolyl-4)-2 (t-butoxycarbonyl-1 éthyl-1 oxyimino)-2 acétique isomère syn.

Par le même traitement on obtient le produit attendu.

Spectre de RMN:

1H à 8,75 ppm (NH-trityle, S) — 1H à 8,50 ppm (NH—CO, M) — 15 H à 7,27 ppm (H trityle, S) — 1H à 6,75 ppm (H thiazole, 2S) — 1H à 5,90 ppm ($H_7$, M) — 1H à 4,99 ppm ($H_6$, D, J=4 Hz) — 3H à 4,55 ppm (—CH—$CH_3$, +$CH_2Br$, M) — 2H à 3,75 ppm ($CH_2S{\rightarrow}O$, M) —

9H à 1,47 ppm (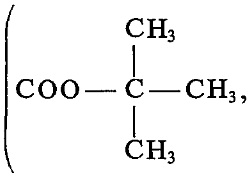, S) – 9H à 1,34 ppm (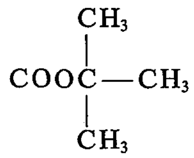, S).

c) Bromure de [(tritylamino-2 thiazolyl-4)-2 (t-butoxycarbonyl-1 éthyl-1 oxyimino)-2 acétamido]-7 (pyridinio-1 méthyl)-3 céphème-3 carboxylate de t-butyle-4 S-oxyde-1 isomère syn

$R_1$ = 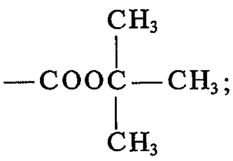; B = 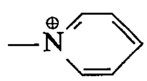 (V)

$R_2 = H$

$R_3 = CH_3$

On opère comme dans l'exemple 3b) à partir du dérivé obtenu ci-dessus.

De la même façon, on obtient le produit attendu.

Spectre de RMN:

3H à 9,05 ppm ($H_2$, pyridine + NH-trityle, M) – 2H à 8,60 ppm ($H_4$, pyridine + NH–CO, M) – 2H à 8,20 ppm ($H_3$, pyridine, M) – 15 H à 7,28 ppm (H trityle, S) – 1 H à 6,82 ppm (H thiazole, 2S) – 1 H à 5,95 ppm ($H_7$, M) – 2H à 5,60 ppm ($CH_2$–$N^+$, S) – 1H à 5,12 ppm ($H_6$, D, J=4 Hz) – 1H à 4,55 ppm (–CH–$CH_3$, Q, J=6 Hz) – 2H à 3,80 ppm ($CH_2$S–O, S.e.) – 9H à 1,45 ppm (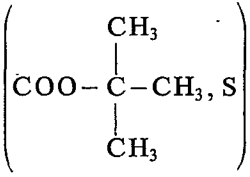, S) – 9H à 1,33 ppm (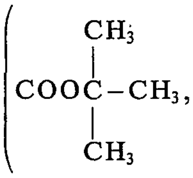, S) – 3H à 1,32 ppm ($CH_3$–CH–, D, J = 6 Hz).

d) CM 40678

On opère comme dans l'exemple 3c) à partir du composé obtenu au paragraphe c).

Spectre de RMN:

4H entre 9 et 11 ppm ($NH_2$, 2 COOH, S. e.) – 2H à 9,0 ppm ($H_2$, pyridine, D, J=6 Hz) – 2H à 8,70 ppm ($H_4$, pyridine + NHCO, M) – 2H à 8,20 ppm ($H_3$, pyridine, M) – 1H à 6,90 ppm (H thiazole, 2S) – 1H à 6,20 ppm ($H_7$, M) – 2H à 5,60 ppm ($CH_2N^+$, S) – 1H à 5,07 ppm ($H_6$, D, J=4 Hz) – 1H à 4,65 ppm (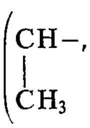, Q, J=6 Hz) – 2H à 3,70 ppm ($CH_2S \rightarrow O$, S.e.) – 3H à 1,33 ppm (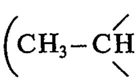, D, J=6 Hz).

En opérant comme dans les exemples 3 ou 4 mais en faisant varier:

– d'une part le dérivé IX de départ

– d'autre part le dérivé de la pyridine utilisé,

on obtient les composés III selon l'invention réunis dans le tableau suivant.

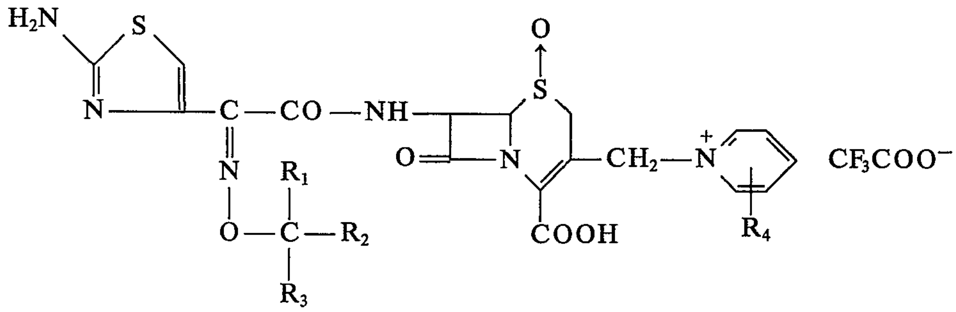

| N. code CM | $R_1$ | $R_2$ | $R_3$ | $R_4$ | Spectre de RMN |
|---|---|---|---|---|---|
| 40420 | COOH | $CH_3$ | $CH_3$ | H | 2H à 9,00 ppm ($H_2$ et $H_6$ pyridine, M) — 1H à 8,50 ppm ($H_4$ pyridine, M) — 3H à 8,20 ppm ($H_3$ et $H_5$ pyridine et NHCO, M) — 4H entre 8 et 10 ppm (2 COOH et $NH_2$) — 1H à 6,70 ppm (H thiazole, S) — 1H à 6,10 ppm ($H_7$, D de D, $J_1 = 9$ Hz), $J_2 = 4$ Hz) — 2H à 5,55 ppm ($CH_2N$, S) — 1H à 5,0 ppm ($CH_2$ en 2, AB, $J_{AB} = 17$ Hz) — 6H à 1,40 ppm 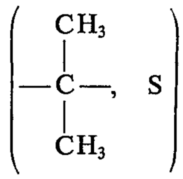 . |
| 40474 | COOH | $-(CH_2)_3-$ | | H | 2H à 9,10 ppm ($H_2$, pyridine, S. e.) — 2H à 8,66 ppm (NH—CO et $H_4$, pyridine M) — 2H à 8,15 ppm ($H_3$, pyridine, S. e.) — — 4H à 7,30 ppm (H échangeables, S. e.) — 1H à 6,80 ppm (H thiazole, S) — 1H à 5,93 ppm ($H_7$, D de D, $J_1$=9 Hz, $J_2$=4,5 Hz) — 2H à 5,53 ppm ($CH_2-\overset{\oplus}{N}<$, S. e.) — 1H à 5,02 ppm ($H_6$,D, J = 4,5 Hz) — 2H à 3,63 ppm ($CH_2S \rightarrow O$, S. e.) — 6H entre 1,5 et 2,6 ppm (cyclobutyle, M) |
| 40475 | COOH | $-(CH_2)_4-$ | | H | 2H à 9,1 ppm ($H_2$, pyridine, S. e.) — 2H à 8,6 ppm (NHCO, $H_4$, pyridine, M) — 2H à 8,20 ppm ($H_3$, pyridine, S. e.) — 5H vers 7,30 ppm (H échangeables, S. e.) — 1H à 6,81 ppm (H thiazole, S) — 1H à 6,03 ppm ($H_7$, M) — 2H à 5,6 ppm ($CH_2\overset{\oplus}{N}<$, S. e.) — 1H à 5,0 ppm ($H_6$, M) — 2H à 3,70 ppm $CH_2S \rightarrow O$, S. e.) — 8H entre 1,3 et 2,5 ppm (cyclopentyle, M). |
| 40582 | COOH | $-(CH_2)_3-$ | | $-4\ CONH_2$ | 2H à 9,13 ppm ($H_2$, pyridine, M) — 2H à 8,50 ppm ($H_3$, pyridine, M) — 1H à 6,95 ppm (H thiazole, S) — 1H à 6,10 ppm ($H_7$, M) — 2H à 5,65 ppm ($CH_2\overset{\oplus}{N}<$, S. e.) — 1H à 5,10 ppm ($H_6$, D, J = 4 Hz) — 6H à 2,3 ppm (cyclobutyle, M). |

Fortsetzung

| N. code CM | $R_1$ | $R_2$ | $R_3$ | $R_4$ | Spectre de RMN |
|---|---|---|---|---|---|
| 40685 | COOH | $-(CH_2)_3$ | | −3 COOH | 1H à 9,51 ppm ($H_2$, pyridine, S. e.) – 2H à 9,10 ppm ($H_4$, et $H_6$, pyridine, M) – 1H à 8,30 ppm ($H_5$, pyridine, M) – 1H 6,80 ppm (H, thiazole, S) – 1H à 6,03 ppm ($H_7$, M) – 2H à 5,66 ppm ($CH_2\overset{\oplus}{N}\leqslant$, S. e.) – 1H à 5,05 ppm ($H_6$, D, J = 4 HZ) – 1H à 3,85 ppm ($CH_2S \rightarrow O$, A de AB, $J_{AB}$ = 17 Hz) – 1H à 3,55 ppm ($CH_2S \rightarrow O$, B de AB, $J_{AB}$ = 17 Hz) – 6H entre 1,6 et 2,6 ppm (cyclobutyle, M). |
| 40801 | COOH | $CH_3$ | $CH_3$ | 3-OH | 3H entre 8,25 et 8,9 ppm (M, CONH, $H_2$, et $H_6$, pyridinium) – 2H à 7,92 ppm (S. e., $H_4$, et $H_5$, pyridinium) – 3H à 7,30 ppm (S. e., $NH_2$) – 1H à 6,80 ppm (S, H thiazole) – 1 OH entre 4,8 et 6,5 ppm (OH, COOH et eau d'hydratation) – 1H à 5,95 ppm (M, $H_7$) – 2H à 5,5 ppm (S. e. $CH_2\overset{+}{N}$) – 1H à 4,97 ppm (D, J = 4 Hz, $H_6$) – 2H à 3,55 ppm (S. e. $CH_2S \rightarrow O$) – 6H à 1,42 ppm 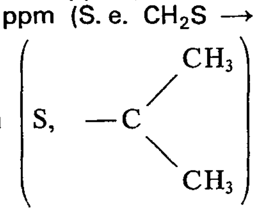 S, |
| 40971 | COOH | $-(CH_2)_3$ | | 3-OH | 1H à 8,70 ppm (D, J = 9 Hz, NHCO) – 2H à 8,45 ppm (M, $H_2$, et $H_6$, pyridinium) – 2H à 7,95 ppm (M, $H_4$, et $H_5$, pyridinium) – 5H entre 6,2 et 7,0 ppm ($NH_2$, OH, 2 COOH) – 1H à 6,80 ppm (S, H thiazole) – 1H à 6,00 ppm (D de D, $J_1$ = 9 Hz, $J_2$ = 4 Hz, $H_7$) – 2H à 5,45 ppm (S. e., $CH_2N$) – 1H à 5,0 ppm (D, J = 4 Hz, $H_6$) – 2H à 3,55 ppm (M, $CH_2S \rightarrow O$) – 6H entre 1,5 et 2,6 ppm (M, 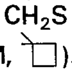). |

Les composés de la présente invention ont été étudiés en ce qui concerne leur activité pharmacologique et plus particulièrement leur activité bactériostatique.

Les composés de formula III possèdent une activité remarquable sur les bactéries gram négatives et spécialement sur les souches productrices de $\beta$-lactamases.

En particulier les composés III sont extrêmement actifs sur les Enterobacter ce qui est particulièrement surprenant pour des composés appartenant à la classe des céphalosporines.

Les résultats obtenus sur différentes souches d'Enterobacter avec les produits de formule III sont réunis dans le tableau ci-dessous.

L'action bactériostatique in vitro a été déterminée en milieu solide par la méthode des dilutions. Les résultats sont exprimés en concentrations minimales inhibitrices (CMI – $\mu$g/ml).

A titre de comparaison on a ajouté dans le tableau un produit appartenant à la demande de brevet européen n° 5830 et répondant à la formule:

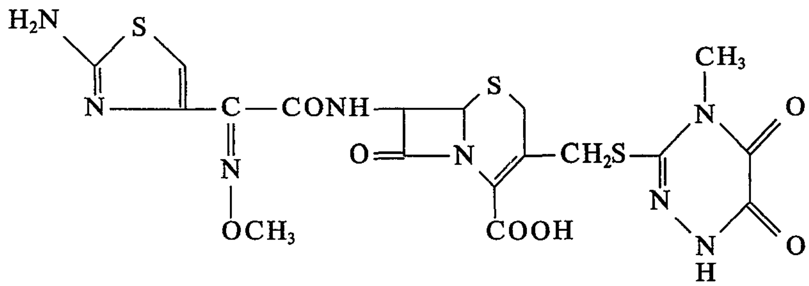

Composé R

Par ailleurs, des essais effectués sur animaux ont montré que les produits de l'invention sont très peu toxiques. De plus des essais réalisés avec les composés CM 40420 et CM 40474 ont prouvé que ces produits étaient dépourvus de néphrotoxicité.

Enfin, des expériences in vivo ont montré une bonne efficacité des produits selon l'invention dans le traitement de maladies expérimentales.

| Produits CM | Souches | | | |
|---|---|---|---|---|
| | Enterobacter P 99 | Enterobacter RO 45 | Enterobacter RO 46 | Enterobacter RL 154 |
| 40365 | 4 | 2 | 8 | 4 |
| 40419 | 2 | 4 | 4 | 4 |
| 40420 | 2 | 1 | 1 | 1 |
| 40471 | 4 | 4 | 8 | 4 |
| 40473 | 2 | 1 | 2 | 1 |
| 40474 | 1 | 0,5 | 0,5 | 0,5 |
| 40475 | 4 | 2 | 2 | 2 |
| 40512 | 4 | 0,5 | 1 | 0,5 |
| 40513 | 4 | 2 | 2 | 2 |
| 40582 | 4 | 4 | 2 | 2 |
| 40678 | 1 | 0,5 | 0,5 | 0,5 |
| 40685 | 128 | 8 | 16 | 4 |
| 40801 | 8 | 2 | 8 | 2 |
| Composé R | 128 | 128 | 256 | 128 |

Les produits de l'invention peuvent donc être utilisés comme antibiotiques en médecine humaine ou vétérinaire. Ils peuvent être utilisés dans toutes les infections bactériennes à germes sensibles.

Les compositions pharmaceutiques sont réalisées à partir des composés (III) sous leur forme acide ou, lorsque leur solubilité est insuffisante, sous forme d'un sel.

Les compositions pharmaceutiques peuvent être solides ou liquides et se présenter par exemple sous forme de comprimés, gélules, granulés, pommades, crèmes, gels ou préparations injectables.

La posologie peut varier dans de larges proportions en particulier suivant le type et la gravité de l'infection à traiter et suivant le mode d'administration. Le plus souvent, chez l'adulte par voie injectable, on

utilise des produits contenant entre 0,250 g et 4 g de produit actif (céphalosporine).

A titre d'exemple de composition pharmaceutique, on peut préparer des ampoules contenant:

| | |
|---|---|
| CM 40474 | 1 g |
| L-lysine | 0,225 g |
| Eau pour préparation injectable | 4 ml |

**Revendications**

1. Céphalosporines S-oxydes de formule

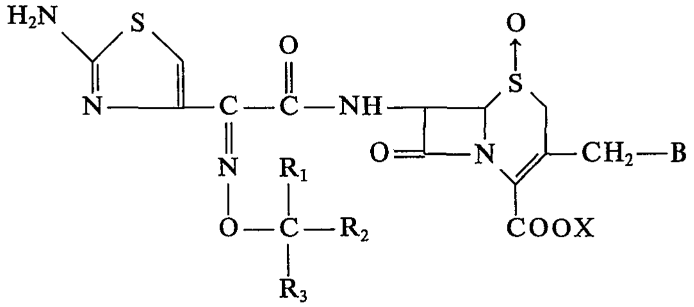

(III)

dans laquelle

$R_1$ représente l'hydrogène ou un groupe COO X',

$R_2$ et $R_3$ qui peuvent être identiques ou différents, représentent chacun l'hydrogène ou un groupe alkyle infériuer ($C_1$—$C_3$)

ou bien

$R_2$ et $R_3$ représentent ensemble un radical 1,3-propylène ou 1,4-butylène,

X et X' qui peuvent être identiques ou différents, représentent de l'hydrogène, un cation ou un ester ou hémiacétate facilement hydrolysable ou métaboliquement labile et pharmaceutiquement acceptables,

B désigne un groupe 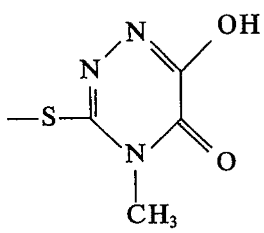

ou encore un groupe pyridinium 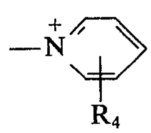

dans lequel $R_4$ représente un hydroxy, un groupe —COOH ou un groupe —CO—$NH_2$.

2. Médicaments antibiotiques, utilisables en médecine humaine et vétérinaire, caractérisés en ce qu'ils contiennent au moins une céphalosporine selon la revendication 1.

3. Médicaments selon la revendication 2, caractérisés en ce qu'ils sont conditionnés pour être utilisés par voie injectable et contenir de 0,25 à 4 g de principe actif (céphalosporine)

4. Procédé pour la préparation des céphalosporines selon la revendication 1 caractérisé en ce que:

– on utilise comme produit de départ un sulfoxyde de formule

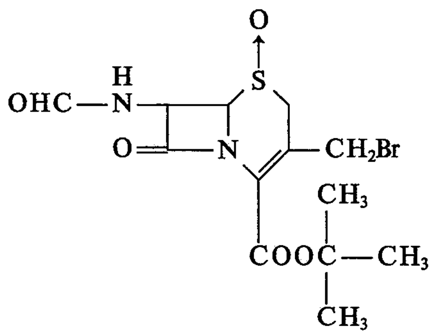

– on fait réagir ce sulfoxyde avec un produit de formule HB dans laquelle B a la signification donnée dans la revendication 1, ladite réaction étant effectuée dans le diméthylformamide en présence d'une base telle que la triéthylamine

– on libère le groupe amino, par action du chlorure de thionyle, de façon à donner naissance au produit de formule

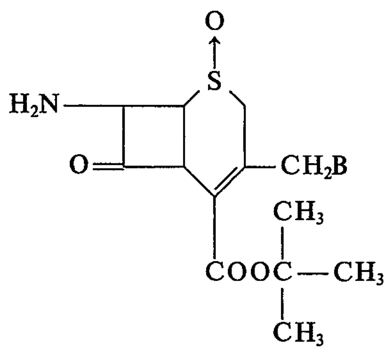

– on effectue l'acylation du produit obtenu à l'aide d'un produit de formule

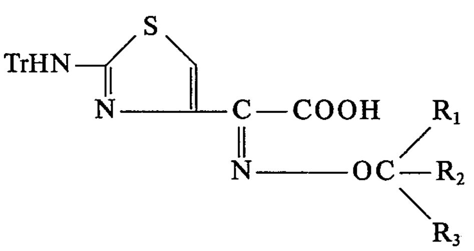

dans lequel le radical Tr est un groupe protecteur du radical amino, par exemple le groupe trityle, le radical acide dudit produit ayant été activé, par exemple par transformation en anhydride

– on élimine, de façon connue, le radical protecteur de l'amine (Tr) et l'ester tertiobutyle du noyau céphème par hydrolyse en milieu acide, de façon à obtenir les produits de formule III dans lesquels X est H

– puis on transforme, si nécessaire, ledit groupe acide pour obtenir les produits dans lesquels X est différent de H.

**Patentansprüche**

1. Cephalosporin-S-oxide der Formel

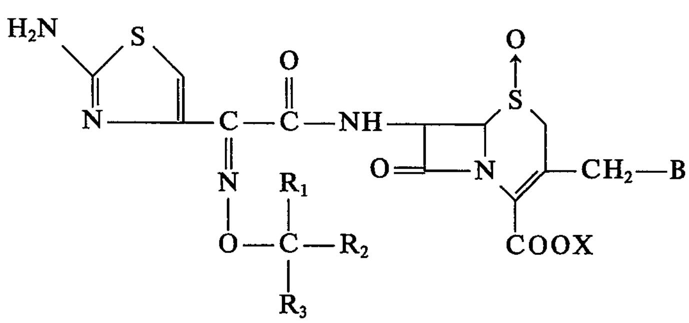

(III)

worin $R_1$ Wasserstoff oder eine COO X'-Gruppe ist, $R_2$ und $R_3$, die gleich oder verschieden sein können, jeweils Wasserstoff oder eine ($C_1$—$C_3$)-Niedrigalkylgruppe darstellen oder aber $R_2$ und $R_3$ zusammen ein 1,3-Propylen- oder 1,4-Butylenradikal darstellen, X und X', die gleich oder verschieden sein können, Wasserstoff, ein Kation oder einen Ester oder ein Hemiacetat, die leicht hydrolysierbar oder metabolisch labil und pharmazeutisch akzeptabel sind, darstellen, B eine Gruppe

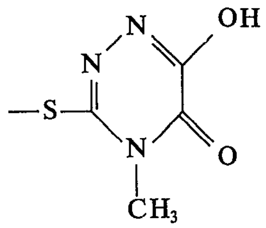

oder auch eine Pyrimidingruppe

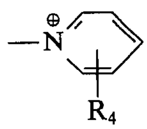

worin $R_4$ Hydroxy, eine —COOH-Gruppe oder eine —CO—$NH_2$-Gruppe darstellen, bezeichnet.

2. In der Human- und Veterinärmedizin verwendbare Antibiotika, dadurch gekennzeichnet, daß sie zumindest ein Cephalosporin nach Anspruch 1 enthalten.

3. Medikamente nach Anspruch 2, dadurch gekennzeichnet, daß sie zur Verwendung auf injizierbarem Wege und mit einem Gehalt von 0,25 bis 4 g Hauptwirkstoff (Cephalosporin) konditioniert sind.

4. Verfahren zur Herstellung der Cephalosporine nach Anspruch 1, dadurch gekennzeichnet, daß

— als Ausgangsprodukt eine Sulfoxid der Formel

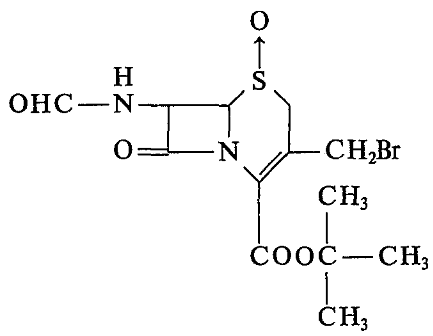

eingesetzt wird,

— dieses Sulfoxid mit einem Produkt der Formel HB, worin B die im Anspruch 1 angegebene Bedeutung hat, reagieren lassen wird, wobei die Umsetzung in Dimethylformamid in Gegenwart einer Base, wir Triäthylamin, durchgeführt wird,

— die Aminogruppe durch Einwirkung von Thionylchlorid freigesetzt wird zur Entstehung eines Produktes der Formel

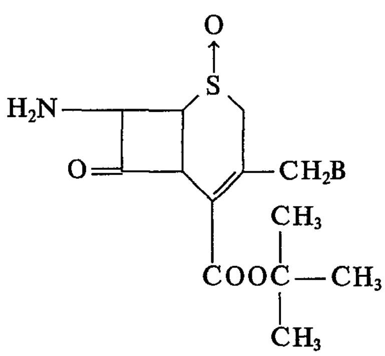

— die Acylierung des erhaltenen Produktes mit Hilfe eines Produktes der Formel

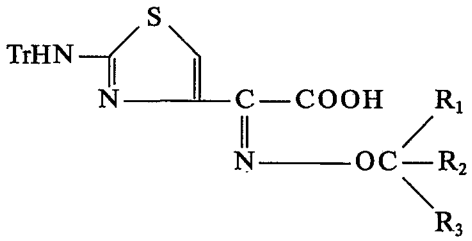

worin das Radikal Tr eine Schutzgruppe des Aminorestes, beispielsweise die Tritylgruppe, ist, wobei der Säurerest des Produktes beispielsweise durch Umwandlung in ein Anhydrid aktiviert wurde, durchgeführt wird,

- auf bekannte Weise die Schutzgruppe des Amins (Tr) und der tert. Butylester des Cephemkerns durch Hydrolyse in saurem Medium derart entfernt wird, daß die Produkte der Formel III, worin X H ist, erhalten werden,
- und anschließend gegebenenfalls zur Erzielung der Produkte, worin X nicht H ist, die Säuregruppe umgewandelt wird.

## Claims

1. Cephalosporines S-oxide with the following formula:

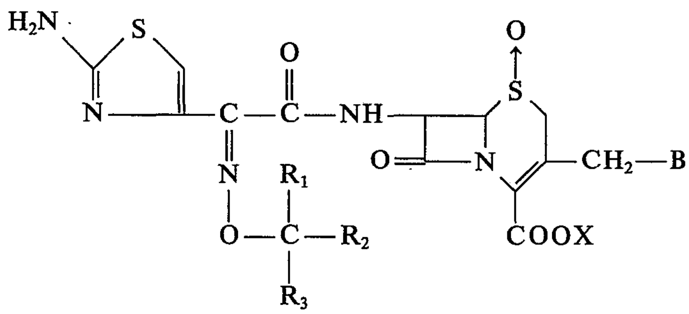

in which

$R_1$ represents hydrogen or a COO X' group,

$R_2$ and $R_3$ which may be identical or different, each representing hydrogen or an inferior alkyl group $(C_1-C_3)$,

$R_2$ and $R_3$ representing together a 1,3-propylene or 1,4 butylene radical,

X and X' which may be identical or different, represent hydrogen, a cation or an ester of an easily hydrolysable or metabolically labile and pharmaceutically acceptable hemiacetate,

B designates a group 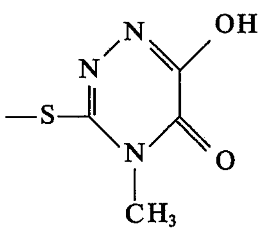

or a pyridinium group 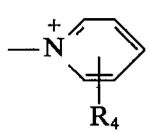

in which $R_4$ represents an hydroxy, an $-COOH$ group or a $-CO-NH_2$ group.

2. Antibiotic medicines for human or veterinary use, characterized in that they contain at least one cephalosporine according to claim 1.

3. Medicines according to claim 2, characterized in that their presentation for use is in the form of injections ranging from 0.25 to 4 g of active ingredient (cephalosporine) per day.

4. Method for preparing cephalosporines, according to claim 1, characterized in that:

- a sulphoxide of the following formula is used as starting product:

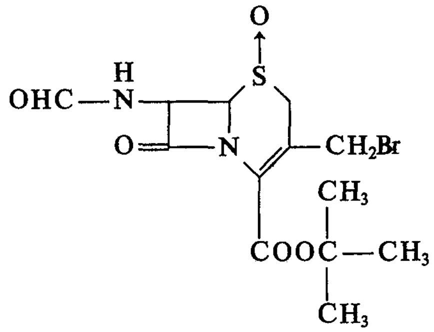

- This sulphoxide is made to react with a product or formula HB in which B has the significance shown below, said reaction being carried out in dimethylformamide in the presence of a base such as triethylamine.
- The amino groups is freed by the action of the thionyl chloride so as to obtain a new product with the following formula:

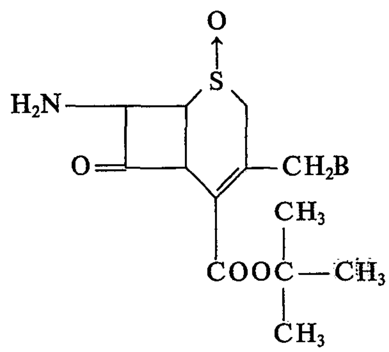

- Acylation of the product obtained is carried out with a product of the following formula:

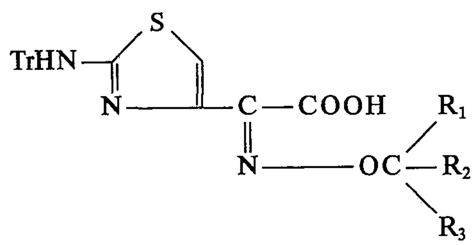

in which the Tr radical is a protecting group of the amino radical, for example, the trityl group, the acid radical of said product having been activated, for instance, by transformation into an anhydride.
- The protecting radical of the amine (Tr) is eliminated by known methods, and the tertiobutyl ester of the cepheme nucleus by hydrolysis in a acid medium in order to obtain the products of formula III in which X is H, then the said acid group is transformed, if necessary, to obtain the products in which X is different from H.